# EUROPEAN PATENT SPECIFICATION

(11) **EP 0 591 999 B1**
(45) Date of publication and mention of the grant of the patent: **23.07.1997**
(21) Application number: 93116350.5
(22) Date of filing: 08.10.1993
(51) Int. Cl.: A61B 17/115

(54) **Surgical fastener applying apparatus**
Chirurgisches Instrument zum Anlegen von Klammern
Instrument d'agrafage chirurgical

(30) Priority: 09.10.1992 US 959275
(43) Date of publication of application: 13.04.1994
(73) Proprietor: United States Surgical Corporation, Norwalk, Connecticut 06856 (US)
(72) Inventor: Viola, Frank J., Sandy Hook, CT 06482 (US); Robertson, John C., Bloomfield, CT 06002 (US)
(74) Representative: Marsh, Roy David

(56) References cited:
- EP-A- 0 503 689
- WO-A-92/03979
- GB-A- 2 016 991
- GB-A- 2 070 499

## Description

### BACKGROUND OF THE INVENTION

### 1. Field of the Invention

The present invention relates to surgical instruments for applying surgical fasteners or staples to body tissue, and more particularly to an apparatus for applying an annular array of surgical staples or fasteners. Still more particularly, the invention relates to an apparatus having an adjustable mechanism for controlling the spacing between the staple pusher member and the anvil member between which the tissue is positioned to effect a circular anastomosis.

### 2. Discussion of the Related Art

Surgical stapling devices for applying an annular array of staples or fasteners to tissue are well known in the art. These devices typically include means for controlling the spacing between the fastener assembly and the anvil member at the distal end of the apparatus. The fastener assembly generally includes a circular array of fasteners such as staples, anastomosis rings, and the like, while the anvil member includes means for completing the circular anastomosis, typically an array of bucket members that clinch the staples after the staples are expelled from the fastener assembly, or may include a locking member for the anastomosis ring. The means for advancing or retracting the anvil in relation to the fastener assembly typically includes a wing-nut type mechanism at a proximal end of the instrument or a rotatable knob member, both of which engage a worm gear arrangement in the handle mechanism to slowly, and methodically advance the anvil member towards the fastener assembly.

Surgical stapling devices for applying an annular array of staples, as well as devices for completing a surgical anastomosis through the provision of anastomosis rings, are well known in gastric and esophageal surgery, for example in classic or modified gastric reconstruction typically formed in an end to end, end to side, or side to side manner. In use, the instrument is positioned within the lumen of an organ such as the stomach, esophagus, or intestine in order to perform the anastomosis. The tissue is positioned between the anvil and the fastener assembly and is typically tied off, for example, by a purse string suture. Thereafter, the anvil member is advanced towards the fastener assembly by rotation of the rotatable knob or wing nut assembly at the proximal end of the instrument to hold the tissue between the anvil member and the fastener assembly. As the staples or the fasteners are expelled from the fastener assembly, a circular knife typically follows the application of the staples to excise unwanted tissue at the anastomosis site. The instrument is then removed from the lumen of the organ.

Closing mechanisms associated with the prior art stapling or fastening devices typically utilize a complex worm gear arrangement or screw bearing member to open and close the spacing between the anvil and the fastener assembly. These devices generally provide a rotatable knob or wing-like assembly remote from the fastener or staple pusher member, and the worm gear mechanism is provided to translate the rotational movement of the knob into longitudinal movement of the anvil member towards the staple pusher member. In order to effect this movement, the surgeon must grasp the device with one hand while rotating the knob or wing-like assembly with the other hand. Because the worm gear is typically a precision component for moving the anvil member in precise increments, it is a time consuming process to move the anvil member a full distance towards the fastener assembly during the surgical procedure.

Typical devices require 15 to 20 full 360° rotations of the knob or wing nut assembly to fully close the instrument in order to fire or expel the staples or fasteners into the tissue.

Apparatuses according to the preamble of claim 1 are known from GB-A-20 70 499, GB-A-20 16 991 and US-A-51 19 983. These known apparatus also comprise knob assemblies or the like as described above to change the position of the anvil member in relation to the staple assembly and therefore also result in the described disadvantages.

A fast closures device for a surgical stapling instrument provided to apply a rectilinear row of staples is known from US-A-51 37 198. The instrument comprises a double four-bar linkage which operates to both place a retaining pin into an anvil so as to maintain proper alignment between a staple cartridge and the anvil and to approximately position the staple cartridge with respect to the anvil. After the approximated positioning of these parts with respect to each other by means of the four-bar linkage mechanism, a gap-setting screw is used so as to finally set the gap between the anvil and the cartridge.

It would be advantageous to expedite the surgical procedure utilizing circular anastomosis instruments. Additionally, although many prior art devices are provided with a visual indicator to signal the surgeon when the anvil has reached a firing position adjacent the staple or fastener assembly, there is generally no indication of the position of the anvil member in relation to the staple assembly until the anvil is immediately adjacent the fastener or staple assembly.

The problems and advantages described above are solved in accordance with the present invention by a surgical stapler apparatus including the features of claim 1. Detailed embodiments of this apparatus are defined in the dependent claims.

The novel surgical stapling or fastening device for performing a circular anastomosis procedure of the present invention provides an instrument having an adjustable closure mechanism for controlling the spacing between the anvil member and the fastener assembly positioned at the distal end of the instrument. The closing mechanism of the present invention provides a mechanism for rapidly approximating the distance between the anvil and the fastener assembly, and further includes a mechanism for incrementally adjusting the remaining distance after the initial rotation. The instrument of the present invention provides a quick and efficient means for approximating the anvil and fastener assembly while including means for accurately positioning the anvil in relation to the fastener assembly to properly set the distance for applying staples or fasteners to the tissue. In addition, the instrument of the present invention allows the surgeon to rapidly move the anvil towards the fastener assembly in a minimal amount of turns so that the surgeon has an indication of the location of the anvil member in relation to the fastener assembly at all times during rotation of the grip member at the handle of the instrument.

### SUMMARY OF THE INVENTION

The present invention provides a surgical device for applying an annular array of fasteners such as staples, rings, or the like having a novel mechanism for adjusting the distance between the movable anvil member and the fastener assembly prior to the application of fasteners to body tissue. The adjustable mechanism controls the spacing between the anvil member and the fastener assembly to advance and retract the anvil member to approximate the distance between the anvil and the fastener assembly prior to the activation of the handle mechanism to expel the fasteners or staples from the fastener assembly. The adjustable closure mechanism comprises an advancing means which approximates the distance between the anvil member and the fastener assembly by providing for an initial movement which moves the anvil member over a large distance and further provides for a subsequent movement which moves the anvil member over a shorter distance. The initial movement allows for coarse adjustment of the distance between the anvil member and the fastener assembly, and the subsequent movement provides for fine adjustment of the distance between the anvil member and fastener assembly. The adjustable closure mechanism of the present invention provides a means for quickly closing the distance between the anvil member and the fastener assembly and further provides means for incrementally adjusting the distance just prior to firing the fasteners from the device.

The adjustable closure mechanism of the present invention may be used with any surgical instrument having a movable anvil member or movable fastener holding member for forming a circular anastomosis positioned at a distal end of the instrument. The adjustable closure mechanism of the present invention expedites the surgical procedure by providing a means for rapidly adjusting the distance between the anvil member and the fastener assembly and further providing a means for fine adjustment of the distance prior to firing. The distance is approximated in a fast and efficient manner to position the anvil in proper alignment for the application of surgical fasteners such as staples, rings or the like.

The apparatus of the present invention comprises a tubular body portion having a staple pusher member disposed at a distal end. The staple pusher member is associated with an annular array of staples and provides means for expelling the staples from the pusher member in response to movement of an actuating means positioned at a proximal end of the tubular body portion. An anvil member is provided opposite the annular array of staples to clinch the staples in the tissue upon expulsion of the staples. The device further comprises means for advancing the anvil member between the extended position away from the staple pusher member and a retracted position adjacent the staple pusher member, where the advancing means moves the anvil member in a two stage advancement. Initial movement of the advancing means advances the anvil member a first distance, while a subsequent movement of the advancing means advances the anvil member a second distance which is less than the first distance caused by the initial movement. The advancing means and the actuating means preferably comprise a handle assembly for the surgical stapler apparatus.

In a preferred embodiment, the means for advancing the anvil member towards the staple pusher member comprises a cam member which is cylindrically shaped and is provided with a helical groove wound about an outer surface of the cam member. The cam member is fixedly secured to an inner rod member which in turn is secured to a flexible member which extends the length of the tubular body portion and terminates at the staple pusher member. The anvil member is preferably removably secured to the flexible member at the staple pusher member.

The handle assembly of the surgical stapler apparatus preferably includes a grip member which extends proximally from the handle assembly and which includes the cam member of the advancing means. The cam member is preferably slidably positioned within the grip member and the helical groove of the cam member is engaged by a pin which is fixedly secured on an inner surface of the grip member. As the grip member is rotated about a longitudinal axis of the apparatus, the pin member rotates with the grip member and causes the helical groove to ride over the pin member to slidably advance and retract the cam member. The slidable movement of the cam member causes the inner rod member to advance and retract, and further causes the flexible member in the tubular body portion to advance and retract. This of course advances and retracts the anvil member to approximate the distance between the anvil member and the staple pusher member.

In the preferred embodiment, a threaded collar member is concentrically positioned about and releasably secured to the cam member, so that the threads of the collar member engage a bushing member which is positioned in the handle assembly. Means for restricting movement of the threaded collar member is provided in the form of pin members which prevent movement of the collar until initial movement of the grip member is completed, i.e. the pin member on the inside surface of the grip member fully travels the helical groove of the cam member and abuts an end of the helical groove. At this time, the retaining pins of the collar member are permitted to drop into countersunk bores in the cam member to remove the restricting means and permit rotation of the threaded collar within the bushing member. The threads of the threaded collar member have a pitch which is less than the pitch of the helical groove, and provides for fine adjustment of the distance between the anvil member and the stapler pusher member to further approximate the anvil member in relation to the staple pusher member.

In use, the grip member is rotated and the helical groove of the cam member rides over the pin on the inside surface of the grip member to rapidly and slidably move the cam member within the grip member. This causes a rapid approximation of the anvil member and rapidly advances the anvil member towards the staple pusher member. Once the pin on the inside surface of the grip member reaches an end of the helical groove, the retaining pins on the threaded collar member are permitted to drop into countersunk bores in the collar and cam member to permit the threaded collar to rotate to further move the anvil member towards the staple pusher member with tissue therebetween. Once the anvil member is a proper distance from the staple pusher member, the surgeon may then fire the staples or fasteners through the tissue to effect a circular anastomosis at that site.

Alternatively, the threaded collar may be eliminated and the cam member provided with a variable pitch so that the initial pitch is greater than the final pitch. This provides for movement of the pin member within the helical groove rapidly during initial rotation of the grip member followed by a fine adjustment stroke during subsequent rotation of the grip member. In a further alternate embodiment, the pin member may be provided on the inner rod member and the helical groove may be positioned on an inner surface of the cam member so that rotation of the grip member moves the inner rod member over the helical groove a large initial distance, and subsequent rotation moves the inner rod member, and consequently the anvil member, shorter distances per rotation of the grip member.

A further embodiment of the present invention provides a grip member having a radially directed slot to accommodate a latch member which is secured to the inner rod member. The latch member is linearly slidable along the grip member to advance and retract the anvil member at the distal end. Fine adjustment of the distance between the anvil member and the staple pusher member is effected by rotation of the grip member when the latch member is fully drawn towards the proximal end of the instrument. Once the latch member is moved to the proximal end of the instrument, the latch member is engaged in a notch in an end knob member which then permits rotation of a cylindrically shaped sleeve member inside the grip member to adjust the final distance between the anvil member and the staple pusher member.

Another embodiment of the present invention provides a grip member which includes at least a pair of telescoping cylinders, one of which is stationary and a second of which is slidably movable in relation to the other cylinder. The movable cylinder is secured to the inner rod member to rapidly advance and retract the anvil member in relation to staple pusher member. Once the initial movement is completed, the movable cylinder, which is further provided with a camming surface at its distal end, is rotatable in relation to the stationary cylinder so that the cam surface engages a bearing surface to provide for fine adjustment between the anvil member and the staple pusher member.

An additional embodiment of the present invention provides a grip member having a lever mechanism which is pivotable in relation to the grip member over the longitudinal axis of the grip member. The lever mechanism rapidly moves the inner rod member, and consequently the anvil member to advance and retract the anvil member in relation to the staple pusher member. Means for fine adjusting the distance between the anvil member and the staple pusher member is provided, and may comprise a rotatable knob member or a ratchet mechanism for fine adjusting the lever mechanism after the initial movement.

In each of the above embodiments, after the fasteners or staples are driven into the tissue, a circular knife positioned at the distal end as part of the staple fastener assembly excises the unwanted tissue and the instrument is quickly removed from the lumen of the organ being repaired. The unwanted tissue is easily removed from the instrument by reversing the advancing means to rapidly move the anvil member away from the staple pusher member.

### BRIEF DESCRIPTION OF THE DRAWINGS

The foregoing features of the present invention will become more readily apparent and may be understood by referring to the following detailed description of an illustrated embodiment of the surgical stapling apparatus and its novel adjustable closure mechanism, taken in conjunction with the accompanying drawings, in which:
Fig. 1 illustrates a perspective view of the surgical stapling apparatus having the preferred adjustable closure mechanism of the present invention;
Fig. 2 illustrates a cross-sectional view of the apparatus of Fig. 1 in which the handle assembly is shown in a position where the anvil member is positioned away from the staple pusher member;
Fig. 3 illustrates a cross-sectional view of the apparatus of Fig. 2 in which an initial movement of the advancing means has been completed;
Fig. 4 illustrates a cross-sectional view of the apparatus of Fig. 2 during a subsequent movement of the advancing means;
Fig. 5 illustrates a cross-sectional view of the apparatus of Fig. 2 after completion of the subsequent movement of the advancing means where the anvil member is positioned adjacent the staple pusher member prior to expelling the staples from the staple pusher member;
Figs. 6a-6c illustrate the advancing means of the apparatus of Fig. 2, in which Fig. 6a illustrates a plan view of the cam member, Fig. 6b illustrates a plan view of the threaded collar member, and Fig. 6c illustrates a cross-sectional view of the threaded bushing;
Fig. 7 illustrates a cross-sectional view of an alternate embodiment of the apparatus of Fig. 2 illustrating the advancing means having a variable helical groove, illustrating the position in which the anvil member is located away from the staple pusher member;
Fig. 8 illustrates a cross-sectional view of the apparatus of Fig. 7 showing the position of the cam member corresponding to the anvil member being positioned adjacent to the staple pusher member;
Fig. 9 illustrates a plan view of the cam member of the apparatus of Figs. 7 and 8;
Fig. 10 illustrates a plan view of the cam member of the apparatus of Figs. 11 and 12;
Fig. 11 illustrates a cross-sectional view of an alternate embodiment of the apparatus of Fig. 7 in which a cam member having a dual pitch helical groove is shown corresponding to the position where the anvil member is located away from the staple pusher member;
Fig. 12 illustrates a cross-sectional view of the apparatus of Fig. 11 in which the cam member is shown in a position corresponding to the anvil member being positioned adjacent to the staple pusher member;
Fig. 13 illustrates a perspective view of an alternate embodiment of the apparatus of Fig. 1;
Fig. 14 illustrates a cross-sectional view of the apparatus of Fig. 13;
Fig. 15 illustrates a perspective view of an alternate embodiment of the apparatus of Fig. 1;
Figs. 16a and 16b illustrate a cross-sectional view of the advancing mechanism of the apparatus of Fig. 15 in which the anvil member is positioned away from the staple pusher member;
Figs. 17a and 17b illustrate cross sectional views of the apparatus of Fig. 15 showing the position of the advancing means corresponding to the anvil member being positioned adjacent to the staple pusher member;
Fig. 18 illustrates a perspective view of an alternate embodiment of the apparatus of Fig. 1;
Figs. 19a and 19b illustrate partial cross-sectional views of the apparatus of Fig. 18 corresponding to a position where the anvil member is located away from the staple pusher member;
Fig. 20 illustrates a partial cross-sectional view of the apparatus of Fig. 18 in which advancing means is in a position corresponding to the anvil member being positioned adjacent to the staple pusher member;
Fig. 21 illustrates a perspective view of an alternate embodiment of the apparatus of Fig. 1;
Figs. 22a-22c illustrate a side schematic views showing the positioning of the lever mechanism of Fig. 21 during actuation;
Figs. 23a and 23b illustrate top schematic views of the advancing means of Fig. 21 before and after actuation;
Fig. 24 illustrates a perspective view of an alternate embodiment of the apparatus of Fig. 21;
Figs. 25a-25c illustrate side schematic views of the lever mechanism of Fig. 24 during actuation; and
Figs. 26a and 26b illustrate top schematic views of the lever mechanism of Fig. 24 before and after actuation.

### DETAILED DESCRIPTION OF THE PREFERRED EMBODIMENTS

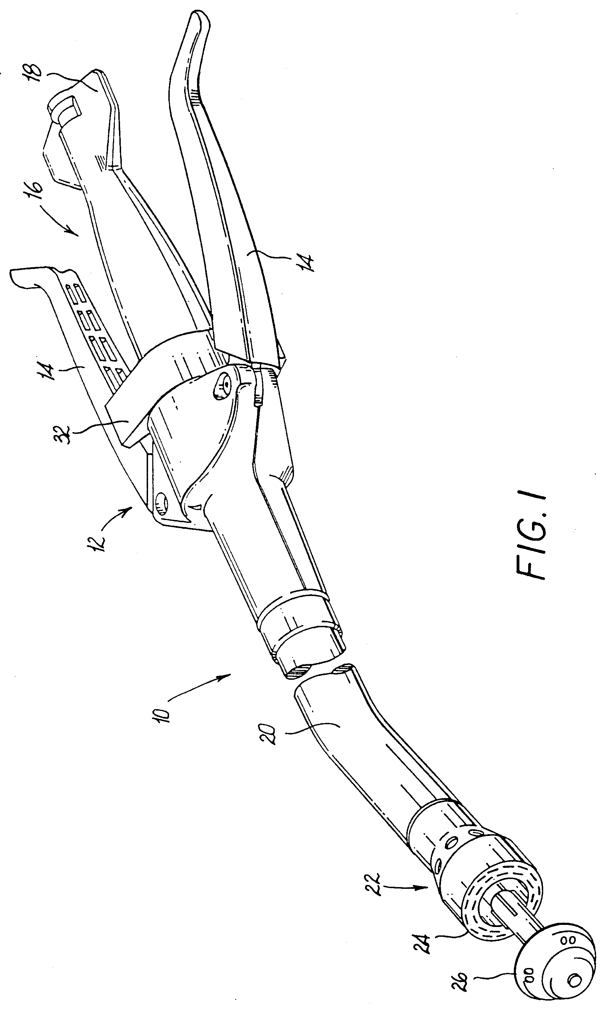

Referring now in specific detail to the drawings, in which like reference numerals identify similar or identical elements throughout the several views, Fig. 1 shows a surgical stapling apparatus 10 which employs the adjustable closure mechanism of the present invention. Apparatus 10 includes a handle assembly 12 having at least one pivotable actuating handle member 14, and further includes advancing means 16. Advancing means 16 preferably comprises a rotatable grip member 18 whose function will be described below.

Extending from handle assembly 12, there is provided a tubular body portion 20 which may be constructed so as to have a curved shaped along its length. Tubular body portion 20 may also be straight, in other embodiments may be flexible to bend to any configuration. Body portion 20 terminates in staple pusher member 22 which is associated with an annular array of staples 24. Positioned opposite staple pusher member 22 is provided an anvil member 26 which is connected to apparatus 10 by shaft 28 at connection means 30. Anvil member 26 and staple pusher member 22 are disclosed in commonly assigned U.S. Patent No. 5,119,983, issued June 9, 1992.

While the preferred embodiment of the present invention utilizes a staple pusher member having an annular array of staples positioned on the tubular body portion, and having the anvil member positioned opposite the staple pusher member for movement towards and away from the staple pusher member, it is of course contemplated that the anvil member may be positioned on the tubular body portion and the staple pusher member and array of staples be positioned opposite the anvil member for movement towards and away from the anvil member. Such a construction is to be considered within the scope of the present invention.

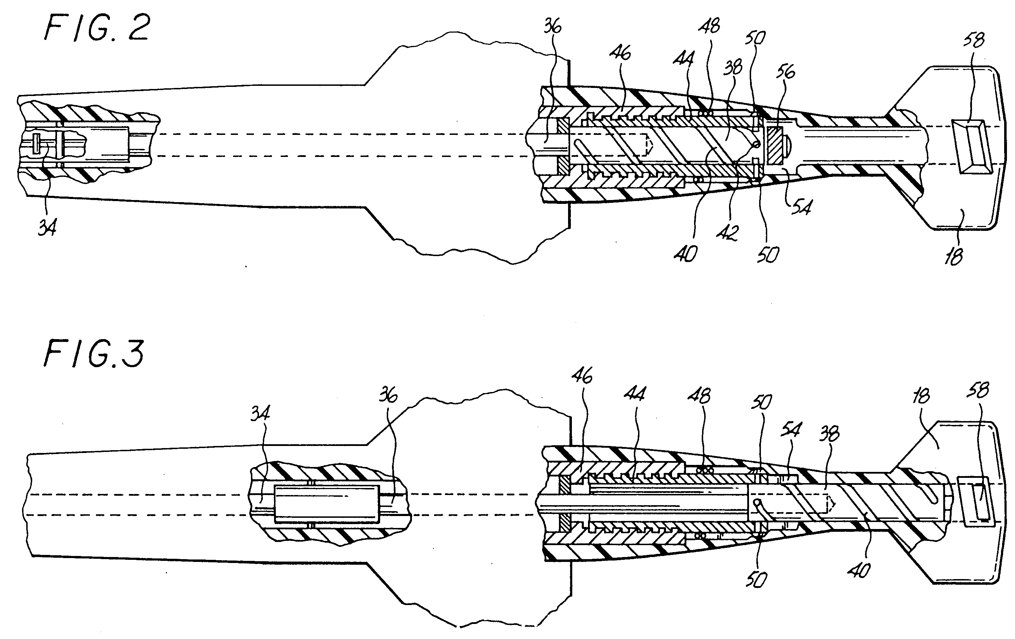

Figs. 2-5 each illustrate a cross-sectional view of the handle assembly 12 of instrument 10 of Fig. 1. Figs. 2-5 illustrate the operation of the advancing means 16 of the adjustable closure mechanism of the present invention. In particular, Fig. 2 illustrates the positioning of the adjustable closure mechanism which corresponds to the location of the anvil member 26 positioned away from the staple pusher member 22.

As seen in Fig. 2, the adjustable closure mechanism includes a cam member 38 having a helical groove 40 positioned thereon. Cam member 38 is secured to handle rod member 36 which extends through handle assembly 12 and is coupled to flexible member 34. Flexible member 34 is positioned within tubular body portion 20 and is coupled to the connection means 30 within staple pusher member 22 for connection to anvil member 26. Movement of inner rod 36 and flexible member 34 controls the advancing and retracting of anvil member 26.

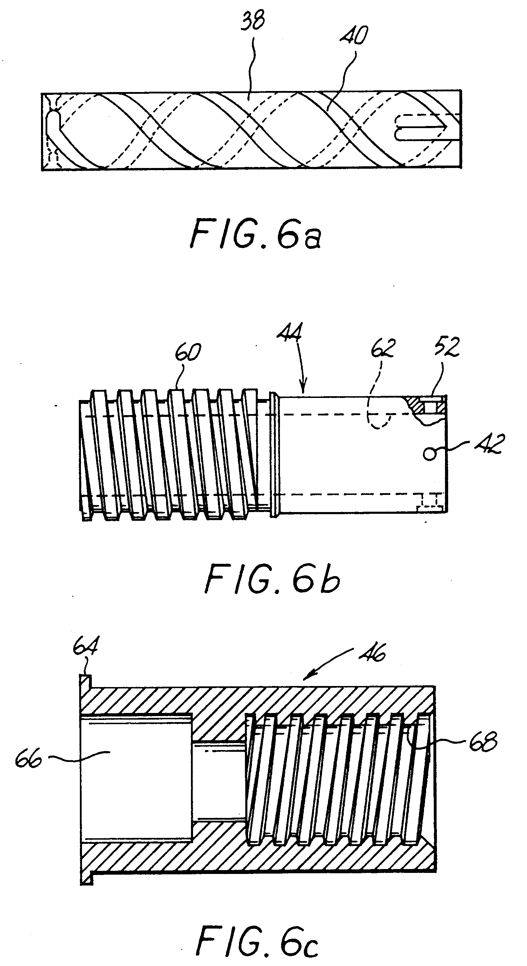

The advancing means essentially comprises grip member 18 having cam member 38, threaded collar 44 and bushing member 46 positioned within grip member 18. Cam member 38 is secured to inner rod member 36 by any suitable means to ensure that movement of cam member 38 operatively advances and retracts inner rod member 36. Grip member 18 includes an inwardly directed pin member 42, through collar 44, which is engaged in helical groove 40 of cam member 38. A spring member 48 is secured about threaded collar member 44, whose operation will be described below. As best seen in Figs. 6a-6c threaded collar 44 is provided with a threaded section 60 which engages the internal threads 68 of bushing member 46. Collar member 44 is provided with an internal passage 62 which slidably accepts cam member 38. Countersink bore holes 52 are provided whose function will be described below. Bushing member 46 also includes an internal passageway 66 and is further provided with a flange member 64 which allows for rotational movement of bushing 46 but prevents longitudinal or axially movement of the bushing.

In operation, the instrument is positioned within a tubular organ in the body of the patient and the ends of the organ to be joined are positioned in the gap between the staple pusher member 22 and the anvil member 26 so that the anvil member 26 is fully extended. As is conventional, the ends of the organ may be secured over the anvil and the staple pusher member by a purse string suture prior to approximation of the anvil member in relation to the staple pusher member. In order to approximate anvil member 26 towards staple pusher member 22, grip member 18 is rotated so that pin member 42 rotates about the longitudinal axis of handle assembly 12. Rotation of pin member 42 causes cam member 38 to begin to move proximally as the helical groove 40 rides over pin member 42. As cam member 38 moves to the position shown in Fig. 3, inner rod member 36 moves proximally with cam member 38, bringing flexible member 34 and anvil member 26 with it. This draws the anvil member 26 into position adjacent staple pusher member 22 and locates the ends of the tissue between these two members. Due to the pitch of helical groove 40, initial rotation of grip member 18 provides for coarse adjustment of the gap or distance between anvil member 26 and staple pusher member 22. The preferred pitch of helical groove 40 allows for approximately two 360_ rotations of grip member 18 to approximate anvil member 26 to its position adjacent staple pusher member 22, when cam member 38 is shown in the position as seen in Fig. 3. Cam member 38 allows for rapid approximation of the anvil member 26, and speeds up the surgical procedure by eliminating as many as ten 360_ turns of the advancing means of a conventional surgical stapling apparatus.

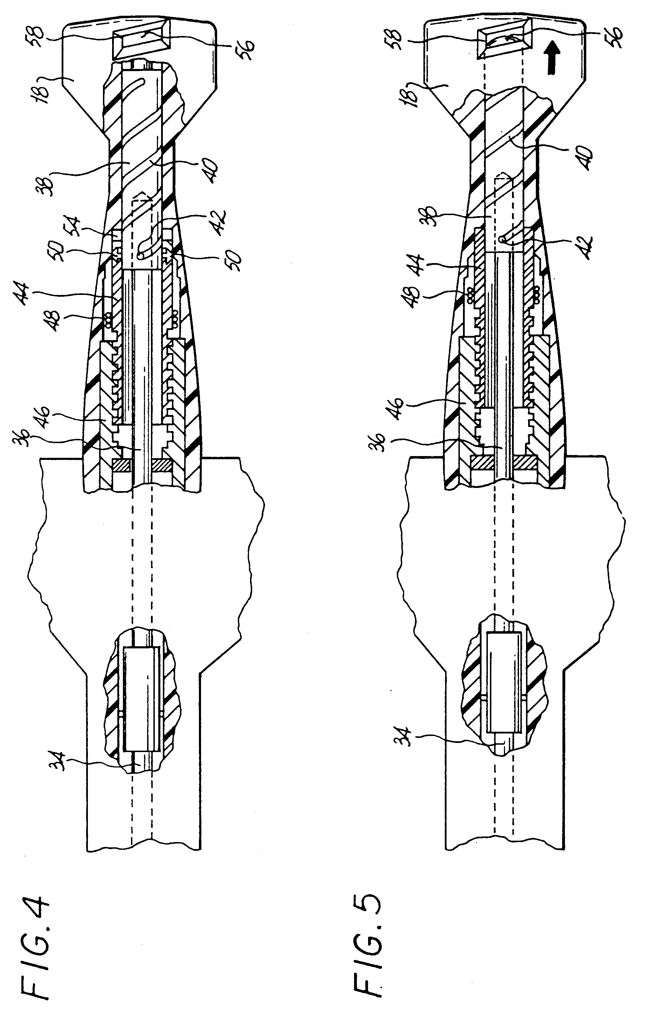

Once the cam member in the position shown in Fig. 3, the advancing means of the present invention provides for fine adjustment of the distance between the anvil member 26 and the staple pusher member 22. When cam member 38 has reached the position shown in Fig. 3, retaining pins 50 are permitted to "drop" into countersunk bore holes 52 in threaded collar 44. When the retaining pins drop into the countersunk bores, it allows threaded collar 44 to begin to advance proximally into bore 54 of grip member 18. As best seen in Fig. 4, continued rotation of grip member 18 tightens spring member 48 about collar 44 to allow collar 44 to begin rotating and sliding in a proximal direction. Bushing 46 is held in place and is allowed only to rotate due to flange 64. As grip member 18 rotates and collar 44 slides rearwardly in bore 54, cam member 38 and inner rod member 36 continued to move rearwardly in a fine adjustable manner due to the threads 60 of threaded collar 44 engaging the inner threads 68 of bushing 46. The precise position of anvil member 26 may be located with respect to staple pusher member 22 by the indicator means on grip member 18, which preferably comprises a colored flag 56 which appears in flag window 58. Once the proper distance is set between anvil member 26 and staple pusher member 22, as shown by the positioning of the advancing means in Fig. 5, and as indicated by the flag 56 being positioned in window 58, interlock means 32 may be released and actuating handles 14 may be pivoted to drive the staples through the tissue against the anvil member to complete the circular anastomosis of the tubular organ.

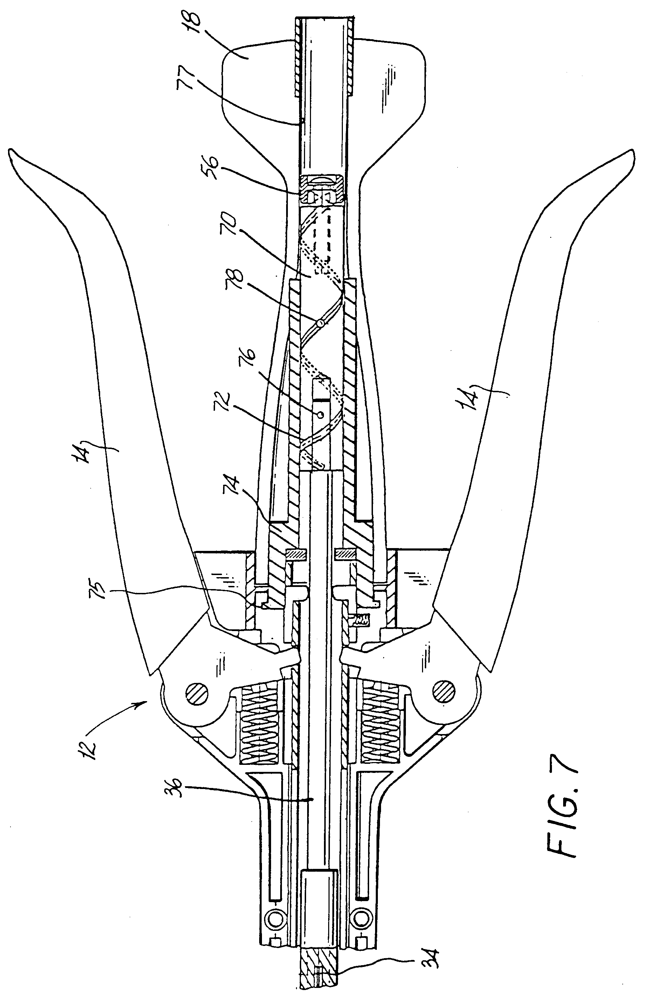

Fig. 7 illustrates the handle assembly 12 having an alternate embodiment of the advancing means of the present invention. As seen in Fig. 7, cam member 70 is provided with a helical groove 72 having a variable pitch which allows for movement of the anvil member 26 over a first distance as well as movement over a second distance which is less than the first distance upon rotation of grip member 18. Fig. 7 illustrates the cam member 70 in a position which corresponds to the anvil member 26 being positioned away from the staple pusher member 22. Cam member 70 is secured to inner rod member 36 in a conventional manner, such as by connection pin 76. Inner rod member 36, as described above, is secured to flexible member 34 which runs through tubular body portion 20. Cam member 70 fits within rotatable sleeve member 74, which will rotate on rotation of grip member 18, but is prevented from moving longitudinally by flange 75. Sleeve member 74 is provided with a rotation pin 78 which engages helical groove 72. The operation of cam member 70 will now be described.

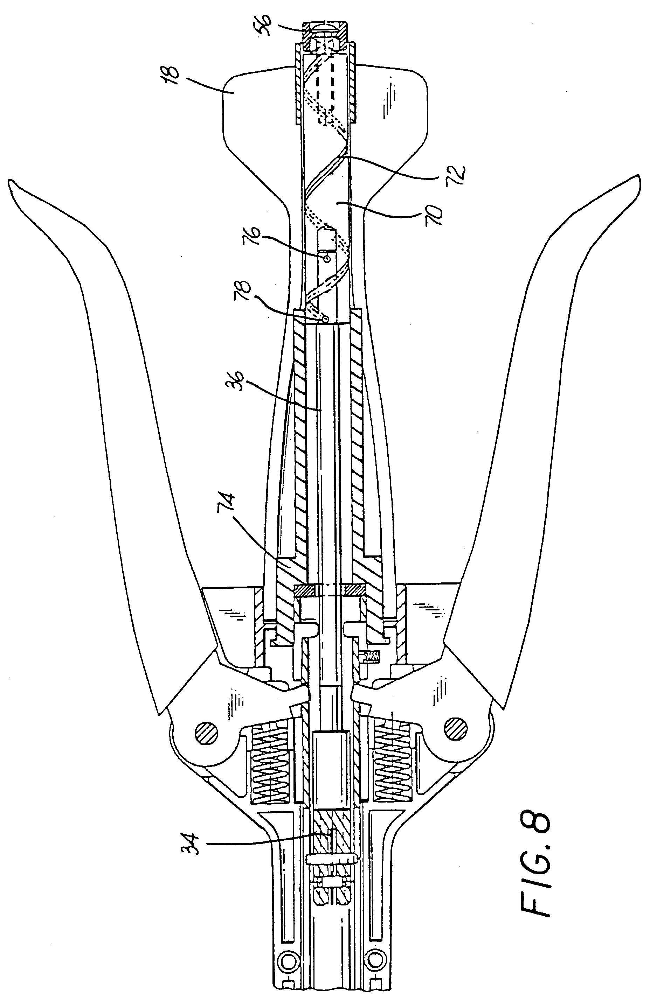

As grip member 18 is rotated, sleeve member 74 also rotates along with rotation pin 78. Rotation pin 78 causes helical groove 72 to ride over the pin 78 drawing cam member, and consequently inner rod member 36, in a proximal direction through bore 77 of grip member 18. As also seen in Fig. 8, rotation pin 78 rides over helical groove 72 at the first pitch to provide for a large approximation of the distance between anvil member 26 and staple pusher member 22. As cam member 70 moves the position shown in Fig. 8, the rotation pin 78 enters the smaller pitch section of helical groove 72, thus providing for fine adjustment of the distance between anvil member 26 and staple pusher member 22. When cam member 70 is in the position shown in Fig. 8, the flag 56 provides visual indication that the anvil member is in a position that will allow firing of the staples through the tissue to perform the circular anastomosis.

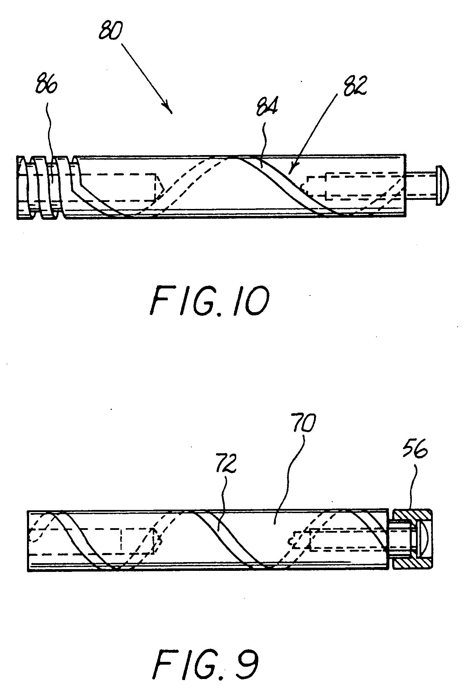

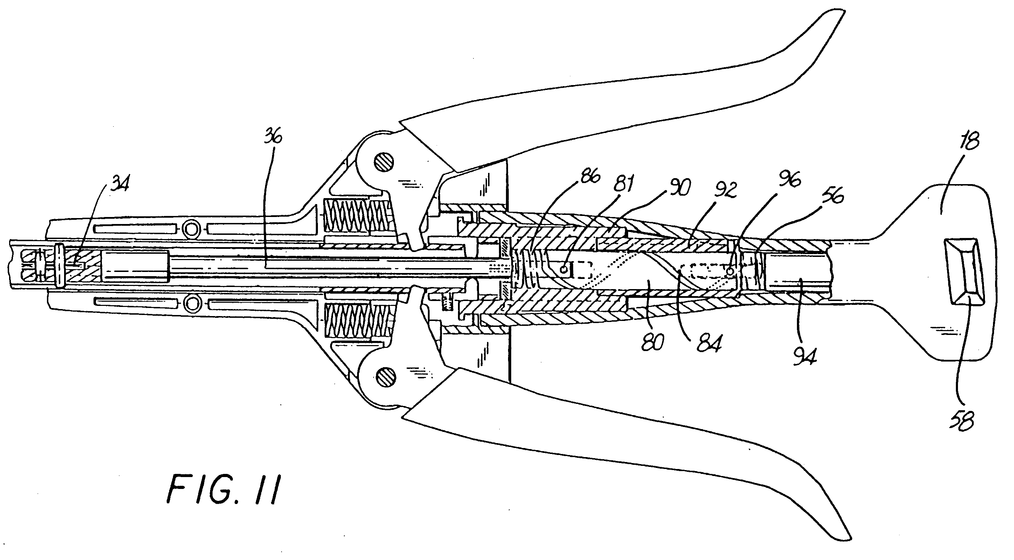

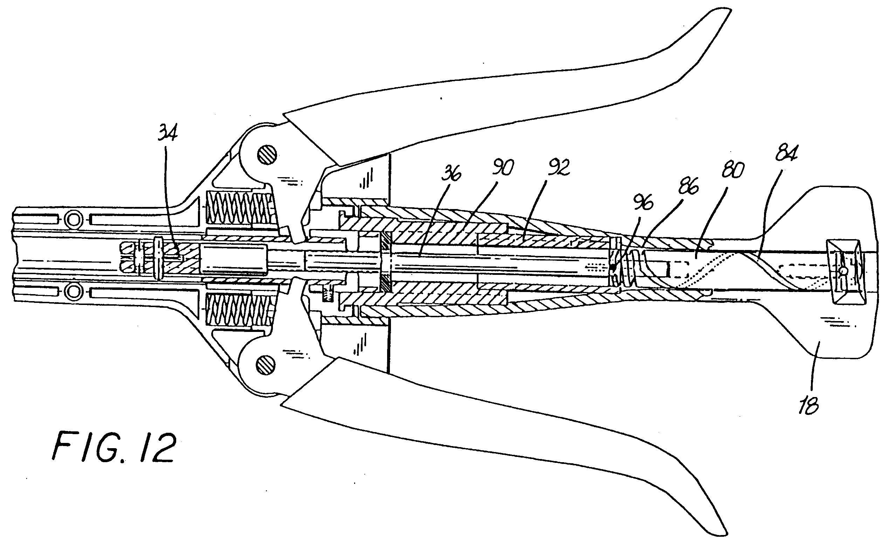

Fig. 9 illustrates cam member 70 as described in Figs. 7 and 8, and Fig. 10 illustrates an alternate embodiment of cam member 80 which is described in relation to Figs. 11 and 12. Cam member 80 is provided with a helical groove 82 having a dual pitch. The first pitch 84 is greater than the second pitch 86, so that first pitch 84 provides for coarse adjustment or a large approximation of the anvil member 26 towards staple pusher member 22, while second pitch 86 provides for fine adjustment or incremental movement of the anvil member 26 towards staple pusher member 22. The operation of cam member 80 is illustrated in Figs. 11 and 12, and is similar to that as described for Figs. 7 and 8 previously.

As seen in Fig. 11, cam member 80 is positioned within rotatable bushing 90 and rotatable sleeve member 92. Cam member 80 is secured to inner rod 36 as described above, such as by pin 81. A rotation pin 96 is provided which is operably secured to rotatable sleeve 92, so that upon rotation of grip member 18, helical groove 82 begins to ride over pin member 96 at first pitch 84. Cam member 80 begins to slide rearwardly in bore 94, thus drawing inner rod member 36 and flexible member 34 in a proximal direction. As cam member 80 reaches a point where rotation pin 96 is at the end of first pitch 84, anvil member 26 is positioned adjacent staple pusher member 22. Further rotation of grip member 18, as seen in Fig. 12, causes second pitch 86 to ride over pin 96 to provide for fine adjustment of the distance between anvil member 26 and staple pusher member 22. Flag 56 appears in flag window 58 to provide a visual indication of the position of anvil member 26 with respect to staple pusher member 22.

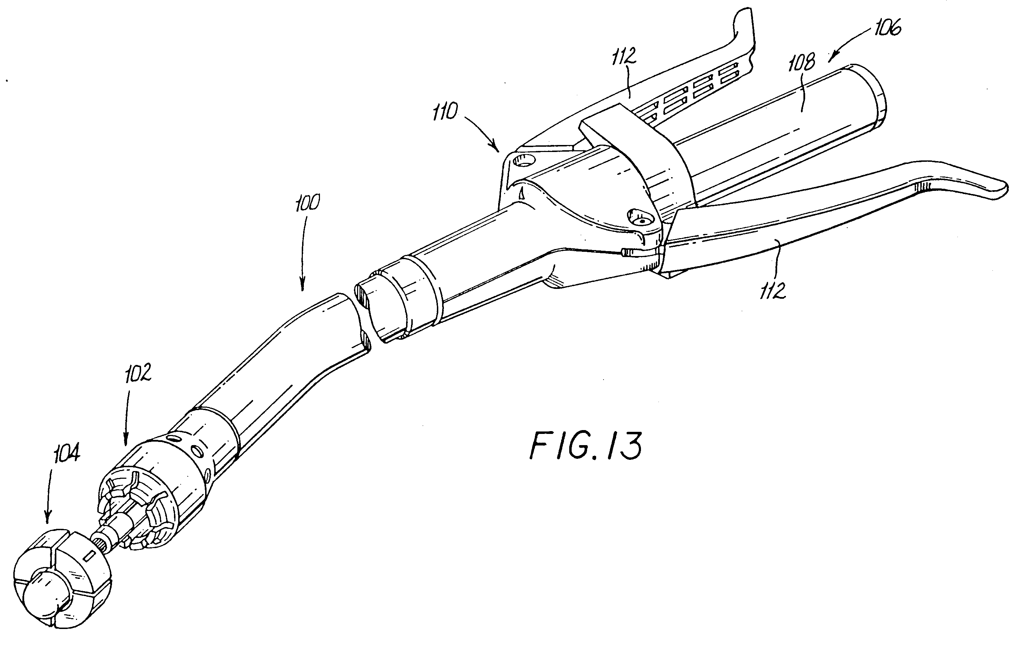

Turning now to Fig. 13, there is illustrated an alternate embodiment of the instrument 100 having the adjustable closure mechanism of the present invention. Instrument 100 includes a fastener assembly 102 which may comprise, for example, an anastomosis ring assembly for engagement with a member 104. Member 104 operates in a manner similar to anvil member 26 described above. Instrument 100 further includes an adjustable closing mechanism 106 which includes a grip member 108 rotatable in a manner described above with respect to grip member 18. Handle assembly 110 further includes actuating handle members 112 which function similar to that described above.

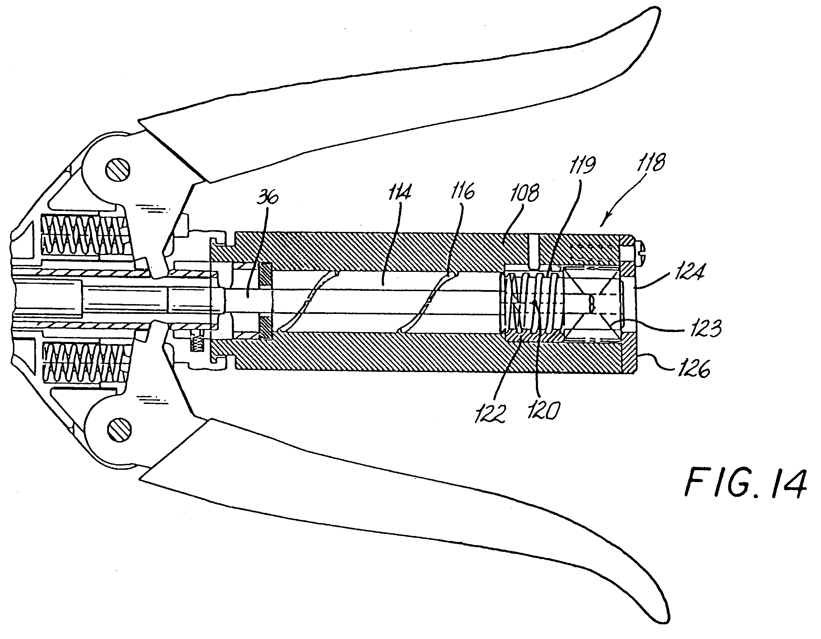

Turning to Fig. 14, there is illustrated a cross-section of the grip member 108 showing the adjustable closure mechanism. Inner rod member 36 is similar to that described above and is secured to advancing means 118 in a conventional manner, such as by pin 120. Advancing means 118 comprises a double threaded collar 119 whose initial position is at the left side of central bore 114 (in relation to Fig. 14). During rotation of grip member 108, double threaded collar 119 begins to ride in a proximal direction over internal threads 116 of bore 114 to the position shown on Fig. 14. Movement of double threaded collar 119 in this direction moves inner rod member 36 and consequently draws member 104 towards fastener assembly 102. Threads 116 engage collar 119 to provide for a coarse adjustment of the distance between fastener assembly 102 and member 104, to rapidly close the distance. Further rotation of grip member 108 causes dual threaded collar 119 to engage threaded bushing 122. As collar 119 engages bushing 122, bushing 122 is forced against spring 123 which results in a short dwell time as threaded collar 119 engages the threads of bushing 122. Bushing 122 provides for fine adjustment of the remaining distance between fastener assembly 102 and member 104. Continued rotation of grip member 108 closes the remaining distance between fastener assembly 102 and member 104, and a visual indication of the distance is provided by opening 124 at end cap 126

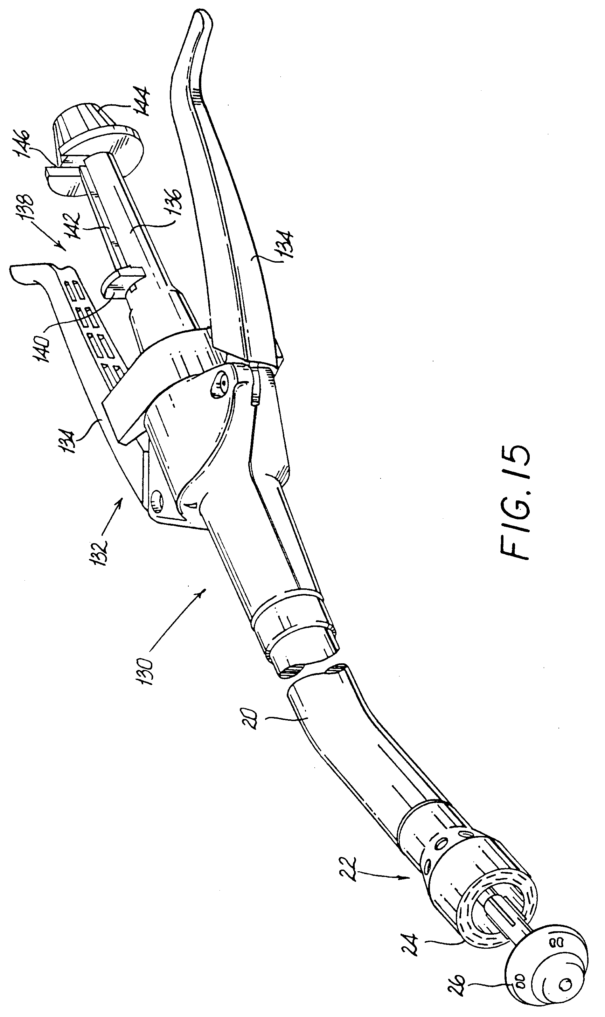

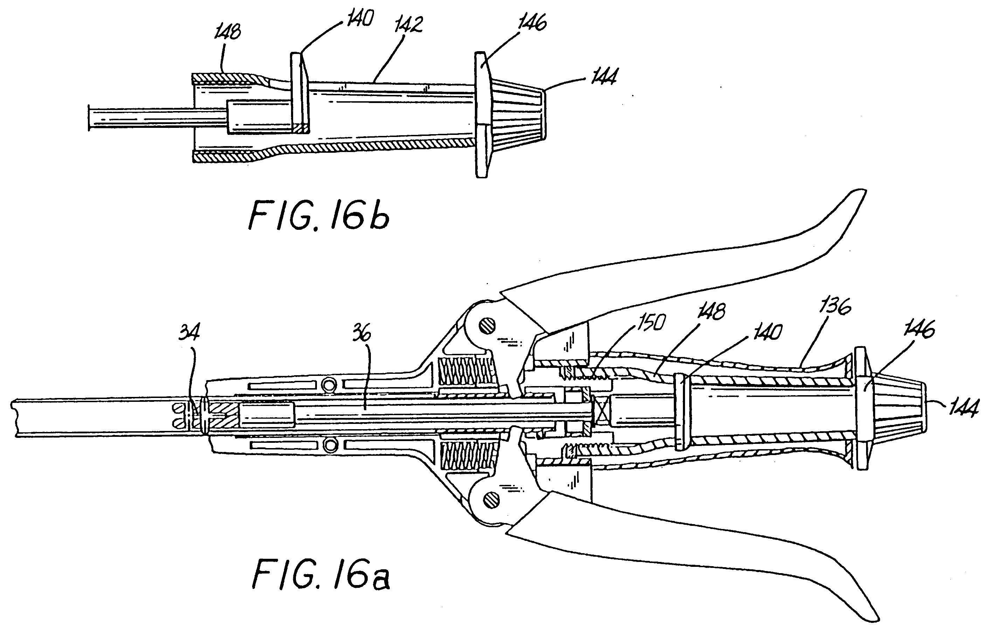

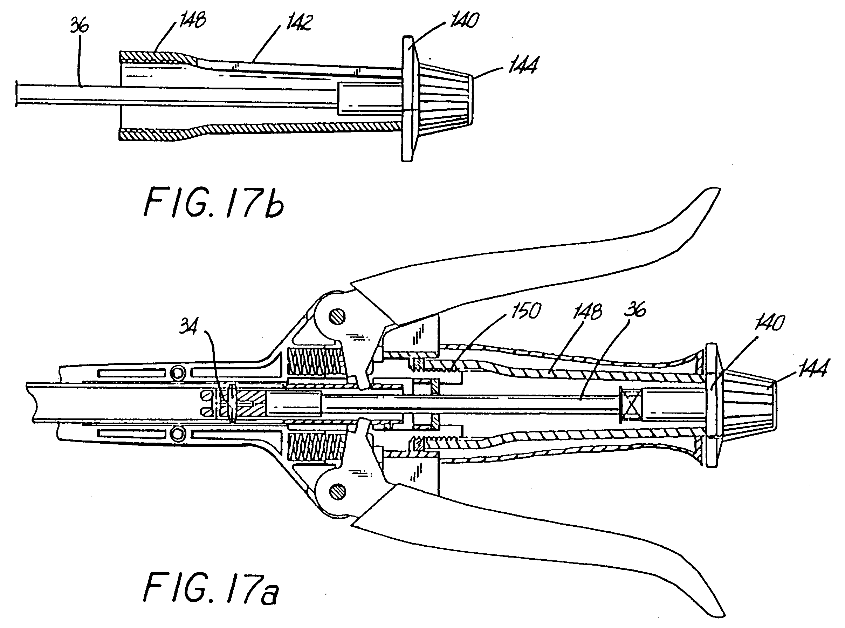

Turning now to Fig. 15, there is illustrated a surgical stapling apparatus having an alternate embodiment of the advancing means 138 of the present invention. Instrument 130 includes handle assembly 132 having pivotable handle members 134 similar to that described above. Extending from handle assembly 132 is tubular body portion 20 which terminates in staples pusher member 22 having anvil assembly 26 positioned adjacent thereto. Advancing means 138 includes a grip member 136 having a linearly slidable latch member 140 which slides along grip member 136 through slot 142. As best seen in Figs. 16 and 17, latch member 140 is fixedly secured to inner rod member 36 which in turn is secured to flexible member 34 similar to that described above. As latch member 140 slides rearwardly towards knob 144, inner rod member 136 slides with it and provides a means for approximating anvil member 26 towards staple pusher member 22 over a large initial distance. As latch member 140 reaches knob 144, it is accommodated in a notch 146 which clears latch 140 from slot 142. As best seen in Figs. 17a and 17b, once latch member 140 clears slot 142, knob 144 may be rotated, which rotates sleeve member 148 about threads 150 to provide for fine adjustment of the remaining distance between anvil member 26 and staple pusher member 22.

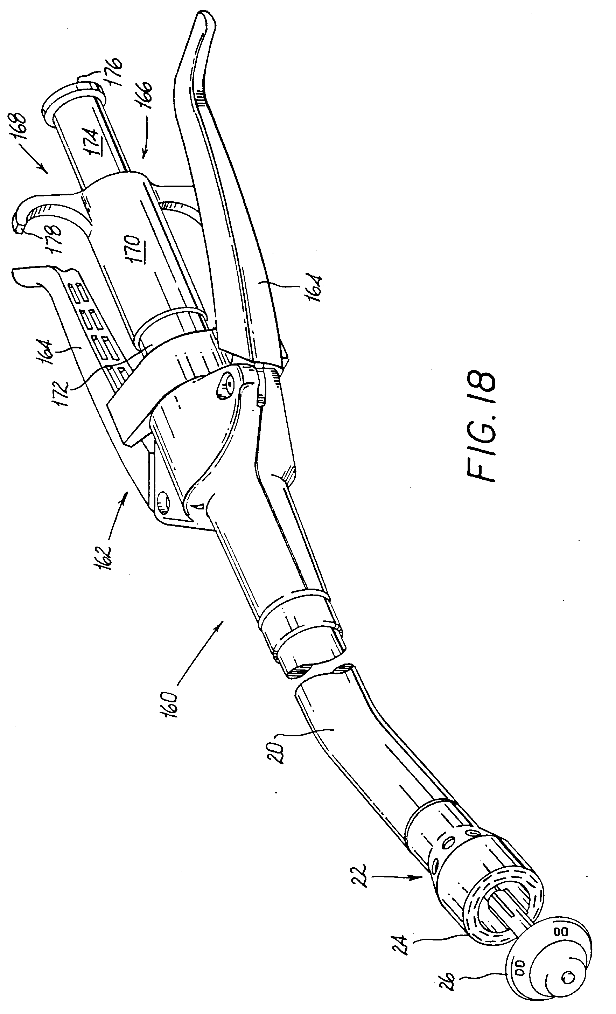

Fig. 18 illustrates a further embodiment of a surgical stapling apparatus having the adjustable closure mechanism of the present invention. Instrument 160 includes handle assembly 162 having pivotable handle members 164 similar to that described above. Extending from handle assembly 162 is tubular body portion 20 which terminates the staple pusher member 22, having anvil member 26 positioned opposite thereto. Handle assembly 162 includes grip member 166 and advancing means 168.

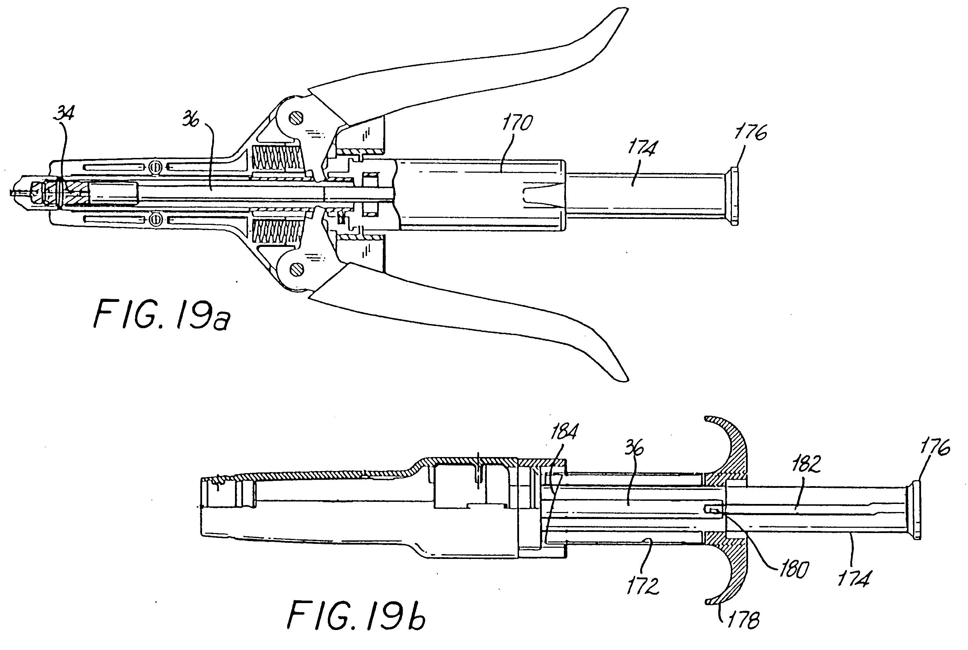

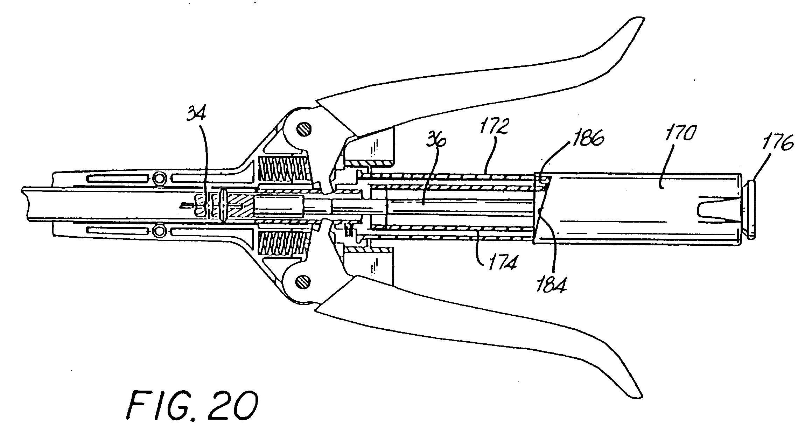

As best seen in Fig. 19, inner rod member 36 is secured to a slidable telescopic outer cylinder 170 which slides over guide cylinder 172 and along guide rod 174. Outer cylinder 170 includes a pin 180 which slides along track 182 of guide rod 174. Outer cylinder 170 slides along guide rod 174 until it reaches stop member 176. As best seen in Fig. 20, once outer cylinder 170 reaches stop member 176, outer cylinder 170 may be rotated so that cam surface 184 rides along bearing surface 186 to provide for fine adjustment of the distance between anvil member 26 and staple pusher member 22.

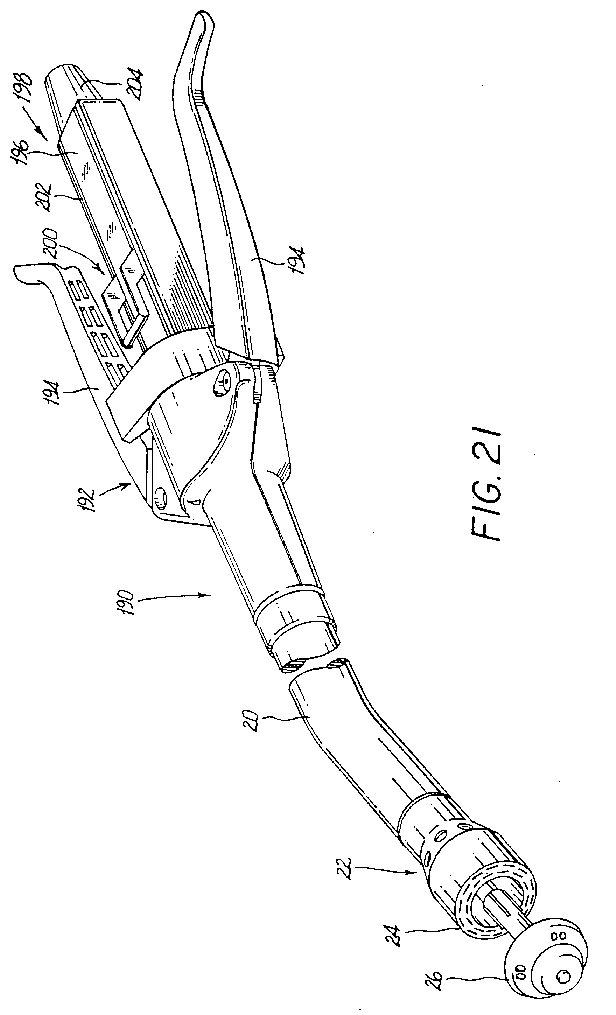

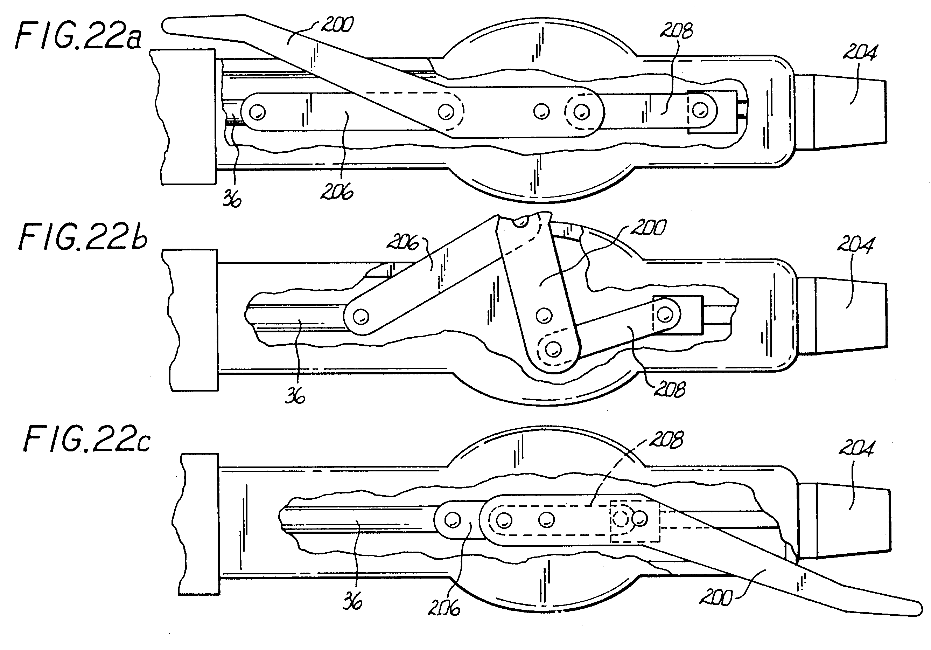

Turning now to Fig. 21, there is illustrated a surgical stapling instrument having an alternate embodiment of the adjustable closure mechanism of the present invention. Instrument 190 includes a handle assembly 192 which includes pivotable handle members 194 similar to that described above. Extending from handle assembly 192 is tubular body portion 20 which terminates in staple pusher mechanism 22 and includes anvil member 26 opposite staple pusher member 22. Handle assembly 192 includes a grip member 196 having advancing means 198 positioned thereon. Advancing means 198 essentially comprises a lever mechanism 200 which is pivotable over a longitudinal axis of the instrument as best seen in Figs. 22a-22c. Knob member 204 is also part of advancing means 198.

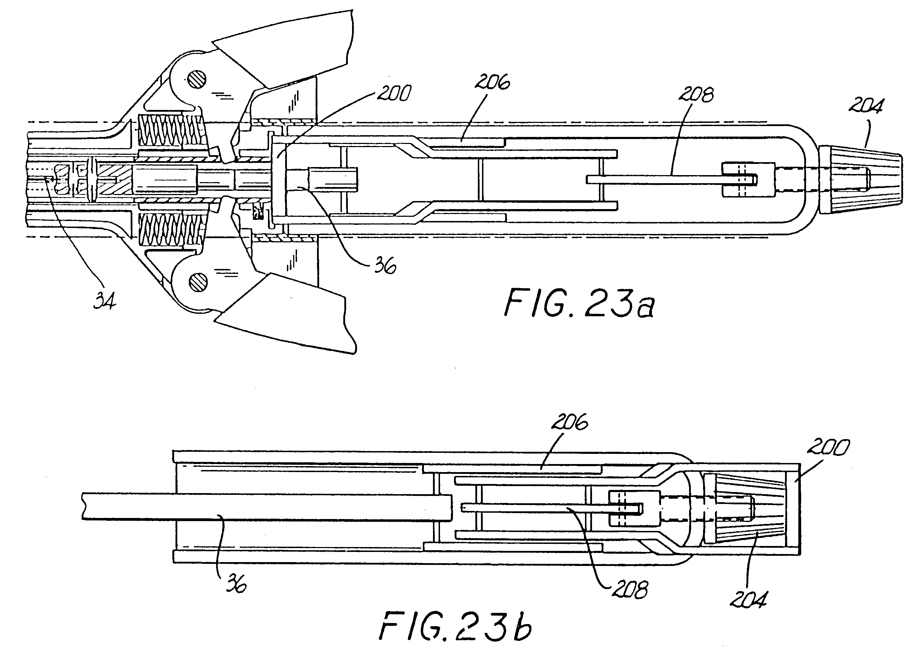

As best seen in Figs. 22a-22c, advancing means 198 comprises lever 200 and includes linkage mechanism 206 and 208. Linkage mechanism 206 is secured to inner rod member 36 for approximating the distance between anvil member 26 and staple pusher member 22. In use, lever 200 is pulled rearwardly as shown in Fig. 22b to move inner rod member 36 in a proximal direction. Lever 200 is pivoted over the longitudinal axis of the instrument and provides for coarse adjustment of the distance between anvil member 26 and staple pusher member 22, as best seen in Fig. 22c. Knob 204 may then be rotated to provide for fine adjustment of the distance between anvil member 26 and staple pusher member 22. Figs. 23a and 23b further show a schematic view of the lever and linkage mechanism.

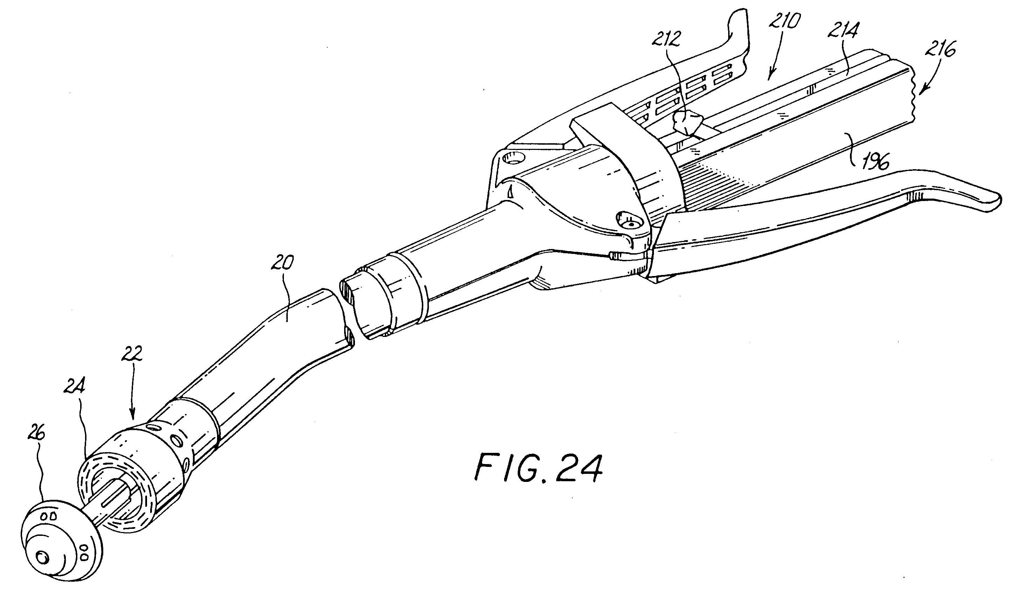

Fig. 24 illustrates an alternate embodiment of the lever mechanism comprising the advancing means of Fig. 21. Fig. 24 illustrates advancing means 210 which includes a lever mechanism 212 which rides a slot 214 in grip member 196. Grip member 196 further includes a ratchet means 216 whose function will be described below.

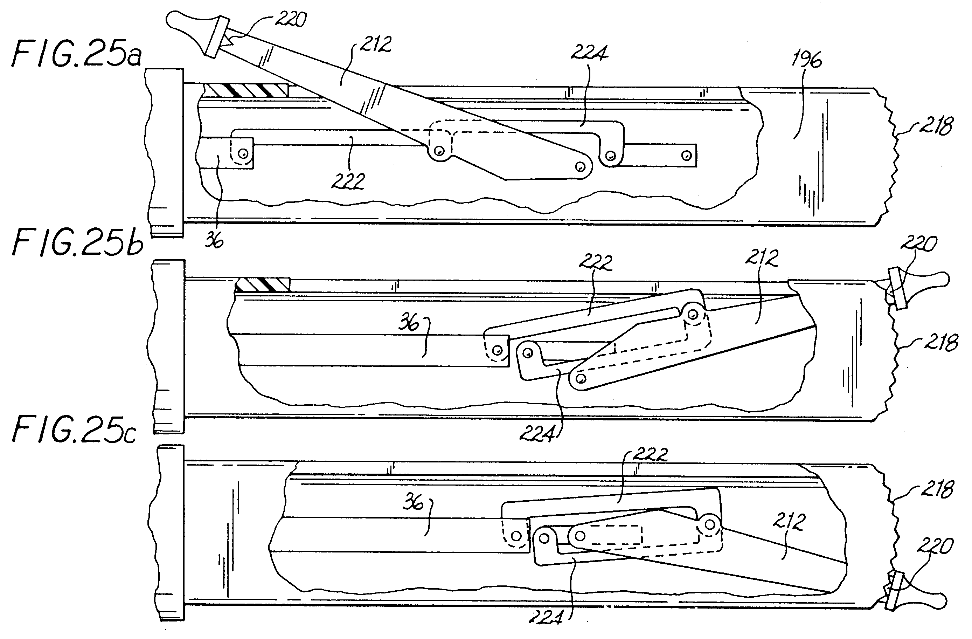

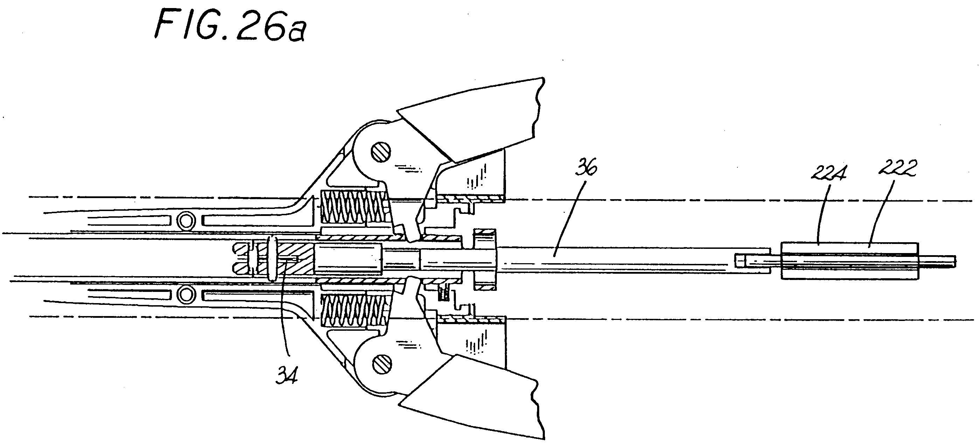

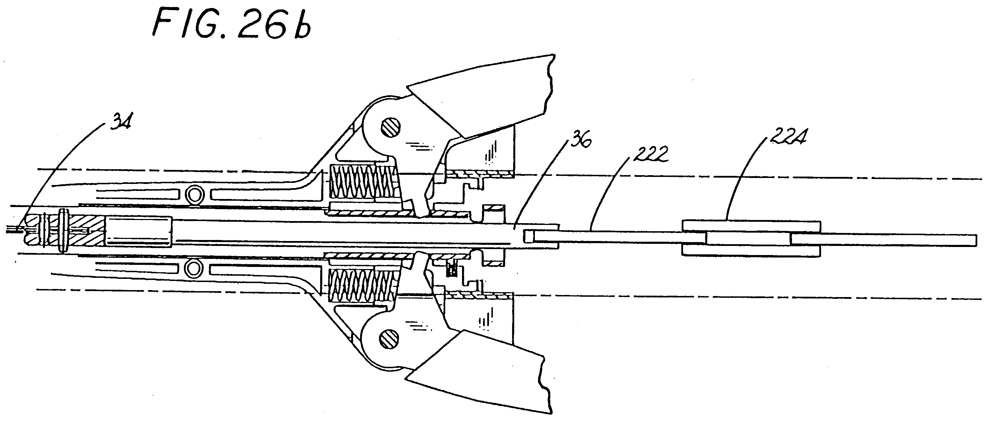

Turning now to Figs. 25a-25c, there is shown the movement of lever mechanism 212. Lever mechanism 212 is operably connected to inner rod member 36 by linkage member 222. Advancing means 210 also includes linkage member 224. Lever mechanism 212 is pivoted over the longitudinal axis of grip member 196 in a manner similar to that described above in relation to Fig. 21. As lever mechanism 212 reaches the position shown in Fig. 25b, pawl detent 220 engages rack member 218 to provide for incremental adjustment of the distance between anvil member 26 and staple pusher member 22. Figs. 26a and 26b illustrate a schematic view of the linkage mechanism of the lever mechanism of Fig. 24.

It should be noted that the various closure mechanisms described herein can be utilized in stapling instruments as well as other anastomosis instruments such as those having a ring assembly as shown in Fig. 13.

The surgical stapling of fastening instrument employing the adjustable closure mechanism of the present invention is a device which may be operated to quickly approximate the distance between the anvil member and the staple pusher member, followed by an additional movement to provide for fine adjustment of the distance between the anvil member and the staple pusher member. The device is quick and easy to use and expedites the surgical procedure.

The claims which follow identify embodiments of the invention additional to those described in detail above.

## Claims

1. A surgical stapler apparatus (10) for applying an annular array of staples comprising:
- a tubular body portion (20);
- a staple pusher member (22) disposed at a distal end of said body portion for expelling said annular array of staples;
- means for actuating said staple pusher member to expel said staples;
- an anvil member (26) disposed at a distal end of said body portion and positioned opposite said staple pusher member to clinch said staples in tissue upon expulsion of said staples; and
- means (16) for advancing one of said members from an extended position away from said other member to a position adjacent said other member,
- characterized in that said advancing means are arranged to move said one member in a two-stage rate of advance, namely an initial advancing movement for coarse adjustment of the distance between the anvil member and the staple pusher member and a subsequent advancing movement for fine adjustment of said distance.

2. Apparatus according to claim 1, wherein said tubular body portion includes a flexible member (34) positioned coaxially therein, said flexible member being operatively coupled to said advancing means at a proximal end and to said one member at a distal end.

3. Apparatus according to claim 1 or 2, wherein said staple pusher member is positioned on a distal end of said tubular body portion, and wherein said advancing means moves said anvil member from an extended position away from said staple pusher member to a retracted position adjacent said staple pusher member.

4. Apparatus according to claim 3, wherein said anvil member is removable from said advancing means when said anvil member is in an extended position to position said tissue therebetween, said anvil member being non-removable in said retracted position.

5. Apparatus according to any one of the preceding claims, wherein a handle assembly (12) including at least one pivotable handle member (14) is operatively coupled to said actuating means, and said advancing means is positioned within said handle assembly.

6. Apparatus according to any one of the preceding claims, wherein said advancing means comprises a grip member (18) positioned at a proximal end of said apparatus, said grip member being rotatable about a longitudinal axis of said apparatus to advance and retract said anvil member.

7. Apparatus according to claim 6 as dependent on claim 2, wherein said advancing means further comprises a cam member (38) slidably positioned within said grip member, said cam member being secured to an inner rod member (36) which is connected to said flexible member and movable to advance and retract inner rod member upon rotation of said grip member.

8. Apparatus according to claim 7, wherein said cam member (38) is cylindrically-shaped and is provided with a helically directed groove (40) on an outer surface thereof, said helical groove being engaged by a pin member (42) secured to said grip member, said helical groove riding over said pin member upon rotation of said grip member to advance and retract said cam member and said anvil member.

9. Apparatus according to claim 7 or 8, wherein said pitch which varies along the cylindrical axis.

10. Apparatus according to claim 9, wherein said helical groove includes a first pitch and a second pitch, said first pitch being greater than said second pitch such that rotation of said grip member initially moves said helical groove over said pin member at said first pitch and then subsequently moves said helical groove over said pin member at said second pitch; such that said cam member, said rod member, said flexible member and said anvil member move a greater distance per rotation of said grip member as said helical groove moves over said pin member at said first pitch than at said second pitch.

11. Apparatus according to claims 8, 9 or 10, wherein said advancing means further comprises a threaded collar member (44) secured to said pin member (42) of said grip member (18), said collar member having a cylindrical shape and being disposed concentrically about said cam member, said collar member threadingly engaging a bushing member (46) and including a threaded portion having a pitch which is less than a pitch of said helical groove, such that initial rotation of said grip member advances said cam member until said pin member abuts an end of said helical groove, subsequent rotation of said grip member advancing said cam member and said threaded collar member to further advance said anvil member in relation to said staple pusher member.

12. Apparatus according to claim 11, said collar member including retaining means (48) for preventing rotation of said collar means prior to completion of said initial rotation of said grip member.

13. Apparatus according to claim 11 or 12, wherein said helical groove (40) of said cam member (38) provides for said coarse adjustment and said collar member (44) provides for said fine adjustment.

14. Apparatus according to any one of the preceding claims, wherein said advancing means includes linearly slidable means for effecting said initial movement, and further includes rotatable means for effecting said subsequent movement.

15. Apparatus according to claim 6 or any one of claims 7 to 12 as dependent on claim 6, wherein said grip member includes a slidable latch (140) to advance and retract said anvil member.

16. Apparatus according to claim 15, wherein said latch (140) is fixedly secured to the inner rod member (36), such that movement of said latch member advances and retracts said anvil member.

17. Apparatus according to claim 15 or 16, wherein said latch (140) provides for said coarse adjustment, and said rotatable sleeve member provides for fine adjustment of said gap.

18. Apparatus according to claim 15, 16 or 17 including a knob member (144) at its proximal end, said knob member including means (146) for engaging said latch member, such that said engagement means prevents rotation of said sleeve member and said knob member unless said latch member is engaged with said knob member.

19. Apparatus according to any one of the preceding claims, wherein said advancing means provides said fine adjustment with incremental advancement of said anvil member.

20. Apparatus according to any one of claims 1 to 5, wherein said advancing means (16) comprises a grip member positioned at a proximal end of said apparatus, said grip member including a lever mechanism (200) pivotable over said grip member along a longitudinal axis of said apparatus to advance and retract said anvil member.

21. Apparatus according to claim 20, wherein said lever mechanism (200) is coupled to an inner rod member (36) which extends from a flexible member (34) within and coaxial with the tubular body portion (20) such that pivoting of said lever mechanism advances and retracts said anvil member.

22. Apparatus according to claim 20 or 21, wherein said advancing means further comprises a ratchet assembly (218, 220) for incrementally advancing said anvil member subsequent to advancing of said anvil member by said pivoting of said lever mechanism over said grip member along a longitudinal axis of said apparatus.

23. Apparatus according to claim 22, wherein said ratchet assembly comprises a plurality of notches (218) forming a rack member on an end portion of said grip member and a detent forming a pawl (220) on a portion of said lever mechanism, said detent and notches cooperating to provide for incremental advancement of said anvil member.

## Patentansprüche

1. Chirurgische Klammervorrichtung (10) zum Anbringen einer ringförmigen Reihe von Klammern umfassend:
- einen röhrenförmigen Körperbereich (20);
- ein Klammerschiebeelement (22), das an einem distalen Ende des Körperbereiches angeordnet ist, um die ringförmige Reihe von Klammern auszustoßen;
- eine Einrichtung zum Betätigen des Klammerschiebeelementes, um die Klammern auszustoßen;
- ein Anschlagelement (26), das an einem distalen Ende des Körperbereiches angeordnet und entgegengesetzt dem Klammerschiebeelement positioniert ist, um die Klammern in Gewebe beim Ausstoßen der Klammern umzubiegen; und
- eine Einrichtung (16), um eines der Elemente von einer ausgestreckten Position entfernt von dem anderen Element zu einer Position neben dem anderen Element vorzurücken,
dadurch **gekennzeichnet**, daß
- die Vorrückeinrichtung gestaltet ist, um das eine Element in einer zweistufigen Vorrückrate zu bewegen, nämlich eine anfängliche Vorrückbewegung zur Grobeinstellung des Abstandes zwischen dem Anschlagelement und dem Klammerschiebeelement und einer nachfolgenden Vorrückbewegung zur Feineinstellung des Abstandes.

2. Vorrichtung gemäß Anspruch 1, wobei der röhrenförmige Körperbereich ein biegbares Element (34) umfaßt, das koaxial darin angeordnet ist, wobei das biegbare Element betriebsmäßig mit dem Vorrückmittel an einem proximalen Ende gekoppelt ist und mit dem einen Element an seinem distalen Ende.

3. Vorrichtung gemäß Anspruch 1 oder 2, wobei das Klammerschiebeelement an einem distalen Ende des röhrenförmigen Körperbereichs angeordnet ist und wobei das Vorrückmittel das Anschlagelement von einer ausgestreckten Position vom Klammerschiebeelement entfernt zu einer zurückgezogenen Position neben dem Klammerschiebeelement bewegt.

4. Vorrichtung gemäß Anspruch 3, wobei das Anschlagelement von dem Vorrückmittel entfernbar ist, wenn das Anschlagelement in einer ausgestreckten Position ist, um das Gewebe dazwischen anzuordnen, wobei das Anschlagelement in der zurückgezogenen Position nicht entfernbar ist.

5. Vorrichtung gemäß einem der vorhergehenden Ansprüche, wobei ein Griffaufbau (12) umfassend zumindest ein schwenkbares Griffelement (14) betriebsmäßig mit der Betätigungseinrichtung verbunden ist, und die Vorrückeinrichtung innerhalb des Griffaufbaus angeordnet ist.

6. Vorrichtung gemäß einen der vorhergehenden Ansprüche, wobei die Vorrückeinrichtung umfaßt ein Griffelement (18), das an einem proximalen Ende der Vorrichtung angeordnet ist, wobei das Griffelement um eine Längsachse der Vorrichtung drehbar ist, um das Anschlagelement vorzurücken und zurückzuziehen.

7. Vorrichtung gemäß Anspruch 6, sofern dieser von Anspruch 2 abhängig ist, wobei die Vorrückeinrichtung weiter umfaßt ein Verschiebeelement (38), das verschiebbar innerhalb des Griffelementes angeordnet ist und das an einem inneren Stangenelement (36) befestigt ist, das mit dem biegbaren Element verbunden und bewegbar ist, um das innere Stangenelement beim Drehen des Griffelementes vorzurücken und zurückzuziehen.

8. Vorrichtung gemäß Anspruch 7, wobei das Verschiebeelement (38) zylinderförmig ist und mit einer schraubenförmig gerichteten Nut (40) auf einer äußeren Oberfläche desselben versehen ist, wobei die schraubenförmige Nut in Eingriff ist mit einem Stiftelement (42), das an dem Griffelement befestigt ist und die schraubenförmige Nut über dem Stiftelement beim Drehen des Griffelementes verfährt, um das Verschiebeelement und das Anschlagelement vorzurücken und zurückzuziehen.

9. Vorrichtung gemäß Anspruch 7 oder 8, wobei sich die Steigung entlang der Zylinderachse verändert.

10. Vorrichtung gemäß Anspruch 9, wobei die schraubenförmige Nut eine erste Steigung und eine zweite Steigung aufweist, wobei die erste Steigung größer als die zweite Steigung ist, so daß die Drehung des Griffelementes anfänglich die schraubenförmige Nut über das Griffelement mit der ersten Steigung und dann nachfolgend die schraubenförmige Nut über das Griffelement mit der zweiten Steigung bewegt; so daß das Verschiebeelement, das Stangenelement, das biegbare Element und das Anschlagelement sich eine größere Entfernung pro Umdrehung des Griffelementes bewegen, wenn sich die schraubenförmige Nut über dem Stiftelement mit der ersten Steigung anstelle der zweiten Steigung bewegt.

11. Vorrichtung gemäß Anspruch 8, 9 oder 10, wobei die Vorrückeinrichtung weiter umfaßt ein Gewindehülsenelement (44), das an dem Stiftelement (42) des Griffelementes (18) befestigt ist, wobei das Hülsenselement eine Zylinderform besitzt und konzentrisch um das Verschiebeelement angeordnet ist, wobei das Griffelement in Gewindeeingriff steht mit einem Buchsenelement (46) und umfassend einen mit Gewinde versehenen Bereich mit einer Steigung, die geringer als eine Steigung der schraubenförmigen Nut ist, so daß eine Anfangsdrehung des Griffelementes das Verschiebeelement vorrückt, bis das Griffelement gegen ein Ende der schraubenförmigen Nut stößt, eine nachfolgende Drehung des Griffelementes das Verschiebeelement und das Gewindehülsenelement vorrückt, um das Anschlagelement in bezug auf das Klammerschiebeelement weiter vorzurücken.

12. Vorrichtung gemäß Anspruch 11, wobei das Hülsenelement eine Halteeinrichtung (48) aufweist, um die Drehung des Hülsenelementes vor dem Abschluß der Anfangsdrehung des Griffelementes zu verhindern.

13. Vorrichtung gemäß Anspruch 11 oder 12, wobei die schraubenförmige Nut (40) des Verschiebeelementes (38) für die Grobanpassung sorgt und das Hülsenelement (44) für die Feinanpassung sorgt.

14. Vorrichtung gemäß einem der vorhergehenden Ansprüche, wobei die Vorrückeinrichtung eine linear verschiebbare Einrichtung umfaßt, um die Anfangsbewegung zu bewirken und weiter eine drehbare Einrichtung zum Bewirken der nachfolgenden Bewegung aufweist.

15. Vorrichtung gemäß Anspruch 6 oder einem der Ansprüche 7 bis 12, sofern diese von Anspruch 6 abhängig sind, wobei das Griffelement eine verschiebbare Klinke (140) umfaßt, um das Anschlagelement vorzurücken und zurückzuziehen.

16. Vorrichtung gemäß Anspruch 15, wobei die Klinke (140) fest am inneren Stangenelement (36) befestigt ist, so daß eine Bewegung des Klinkenelementes das Anschlagelement vorrückt und zurückzieht.

17. Vorrichtung gemäß Anspruch 15 oder 16, wobei die Klinke (140) für die Grobeinstellung dient und das drehbare Hülsenelement für die Feineinstellung des Abstandes dient.

18. Vorrichtung gemäß Anspruch 15, 16 oder 17 umfassend ein Knopfelement (144) an ihrem proximalen Ende, wobei das Knopfelement eine Einrichtung (146) umfaßt, um in Eingriff zu treten mit dem Klinkenelement, so daß das Eingriffsmittel eine Drehung des Hülsenelementes und des Knopfelementes verhindert, wenn nicht das Klinkenelement in Eingriff mit dem Knopfelement ist.

19. Vorrichtung gemäß einem der vorhergehenden Ansprüche, wobei das Vorrückmittel für die Feineinstellung mit einer schrittweisen Annäherung des Anschlagelements sorgt.

20. Vorrichtung gemäß einem der Ansprüche 1 bis 5, wobei die Vorrückeinrichtung (16) ein Griffelement umfaßt, das an einem proximalen Ende der Vorrichtung positioniert ist, wobei das Griffelement einen Hebelmechanismus (200) umfaßt, der über dem Griffelement entlang einer Längsachse der Vorrichtung verschwenkbar ist, um das Anschlagelement vorzurücken und zurückzuziehen.

21. Vorrichtung gemäß Anspruch 20, wobei der Hebelmechanismus (200) an ein inneres Stangenelement (36) gekoppelt ist, das sich von einem biegbaren Element (34) innerhalb und koaxial mit dem röhrenförmigen Körperbereich (20) so erstreckt, daß das Verschwenken des Hebelmechanismus das Anschlagelement vorrückt und zurückzieht.

22. Vorrichtung gemäß Anspruch 20 oder 21, wobei das Vorrückmittel weiter umfaßt einen Klinkenaufbau (218, 220), um schrittweise das Anschlagelement nach dem Vorrücken des Anschlagelementes durch das Verschwenken des Hebelmechanismus über dem Griffelement entlang einer Längsachse der Vorrichtung vorzurücken.

23. Vorrichtung gemäß Anspruch 22, wobei der Klinkenaufbau umfaßt eine Vielzahl von Nuten (218), die ein Zahnstangenelement auf einem Endbereich des Griffelementes bilden und einen Anschlag, der eine Klaue (220) auf einem Bereich des Hebelmechanismus bildet, wobei der Anschlag und die Nuten zusammenwirken, um für ein abschnittsweises Vorrücken des Anschlagelementes zu sorgen.

## Revendications

1. Appareil d'agrafage chirurgical (10) pour appliquer une rangée annulaire d'agrafes comportant :
- une portion de corps tubulaire (20);
- un élément poussant les agrafes (22) disposé à une extrémité distale de ladite portion de corps pour expulser ladite rangée annulaire d'agrafes;
- un moyen pour actionner ledit élément poussant les agrafes pour expulser lesdites agrafes;
- un élément d'enclume (26) disposé à une extrémité distale de ladite portion de corps et positionné en face dudit élément poussant les agrafes pour rabattre lesdites agrafes dans le tissu lors de l'expulsion desdites agrafes; et
- des moyens (16) pour faire avancer l'un desdits éléments d'une position étendue au loin dudit autre élément à une position adjacente audit autre élément,
caractérisé en ce que lesdits moyens d'avance sont agencés pour déplacer ledit élément précité suivant une allure d'avance en deux étapes, à savoir un mouvement d'avance initial en vue d'un ajustement grossier de la distance entre l'élément d'enclume et l'élément poussant les agrafes et un mouvement d'avance subséquent en vue d'un ajustement fin de ladite distance.

2. Appareil selon la revendication 1, où ladite portion de corps tubulaire inclut un élément flexible (34) positionné coaxialement dans celle-ci, ledit élément flexible étant accouplé fonctionnellement audit moyen d'avance à une extrémité proximale et audit élément précité à une extrémité distale.

3. Appareil selon la revendication 1 ou 2, où ledit élément poussant les agrafes est positionné à une extrémité distale de ladite portion de corps tubulaire et où ledit moyen d'avance déplace ledit élément d'enclume d'une position étendue au loin dudit élément poussant les agrafes à une position rétractée adjacente audit élément poussant les agrafes.

4. Appareil selon la revendication 3, où ledit élément d'enclume peut être retiré dudit moyen d'avance lorsque ledit élément d'enclume se trouve dans une position étendue pour positionner ledit tissu entre ceux-ci, ledit élément d'enclume ne pouvant pas être enlevé dans ladite position rétractée.

5. Appareil selon l'une des revendications précédentes, où un ensemble de poignée (12) incluant au moins un élément de poignée pivotant (14) est accouplé fonctionnellement audit moyen d'actionnement, et ledit moyen d'avance est positionné dans ledit ensemble de poignée.

6. Appareil selon l'une des revendications précédentes, où ledit moyen d'avance comporte un élément de saisie (18) positionné à une extrémité proximale dudit appareil, ledit élément de saisie pouvant tourner autour d'un axe longitudinal dudit appareil pour faire avancer et rétracter ledit élément d'enclume.

7. Appareil selon la revendication 6 dépendant de la revendication 2, où ledit moyen d avance comporte en outre un élément de came (38) positionné de manière coulissante dans ledit élément de saisie, ledit élément de came étant fixé à un élément de tige intérieur (36) qui est relié audit élément flexible et qui est déplaçable pour faire avancer et rétracter l'élément de tige intérieur lors de la rotation dudit élément de saisie.

8. Appareil selon la revendication 7, où ledit élément de came (38) a une forme cylindrique et présente une rainure hélicoïdale (40) sur sa surface extérieure, et avec ladite rainure hélicoïdale étant mis en prise un élément de cheville (42) fixé audit élément de saisie, ladite rainure hélicoïdale passant sur ledit élément de cheville lors de la rotation dudit élément de saisie pour faire avancer et pour rétracter ledit élément de came et ledit élément d'enclume.

9. Appareil selon la revendication 7 ou 8, où ledit pas varie le long de l'axe cylindrique.

10. Appareil selon la revendication 9, où ladite rainure hélicoïdale présente un premier pas et un deuxième pas, ledit premier pas étant plus grand que ledit deuxième pas de telle sorte que la rotation dudit élément de saisie déplace initialement ladite rainure hélicoïdale sur ledit élément de cheville suivant ledit premier pas et déplace ensuite ladite rainure hélicoïdale sur ledit élément de cheville suivant ledit deuxième pas; de telle sorte que ledit élément de came, ledit élément de tige, ledit élément flexible et ledit élément d'enclume se déplacent sur une plus grande distance à la suite de la rotation dudit élément de saisie que ladite rainure hélicoïdale se déplace sur ledit élément de cheville suivant ledit premier pas que suivant ledit deuxième pas.

11. Appareil selon les revendications 8, 9 ou 10, où ledit moyen d avance comporte en outre un élément de collier fileté (44) fixé audit élément de cheville (42) dudit élément de saisie (18), ledit élément de collier ayant une forme cylindrique et étant disposé concentriquement autour dudit élément de came, ledit élément de collier venant en prise de filetage avec un élément de douille (46) et incluant une portion filetée avec un pas qui est plus petit qu'un pas de ladite rainure hélicoïdale de telle sorte q'une rotation initiale dudit élément de saisie fait avancer ledit élément de came jusqu'à ce que ledit élément de cheville bute contre une extrémité de ladite rainure hélicoïdale, une rotation subséquente dudit élément de saisie faisant avancer ledit élément de came et ledit élément de collier fileté pour faire avancer davantage ledit élément d'enclume relativement audit élément poussant les agrafes.

12. Appareil selon la revendication 11, où ledit élément de collier inclut un moyen de retenue (48) pour empêcher la rotation dudit moyen de collier avant la fin de ladite rotation initiale dudit élément de saisie.

13. Appareil selon la revendication 11 ou 12, où ladite rainure hélicoïdale (40) dudit élément de came (38) permet ledit ajustement grossier, et ledit élément de collier (44) permet ledit ajustement fin.

14. Appareil selon l'une des revendications précédentes, où ledit moyen d'avance inclut un moyen pouvant coulisser linéairement pour effectuer ledit mouvement initial et comporte en outre un moyen tournant pour effectuer ledit mouvement subséquent.

15. Appareil selon la revendication 6 ou l'une des revendications 7 à 12 dépendant de la revendication 6, où ledit élément de saisie comporte un loquet coulissant (140) pour faire avancer et pour rétracter ledit élément d'enclume.

16. Appareil selon la revendication 15, où ledit loquet (140) est fixé solidement à l'élément de tige intérieur (36) de telle sorte que le mouvement dudit élément de loquet fait avancer et rétracte ledit élément d'enclume.

17. Appareil selon la revendication 15 ou 16, où ledit loquet (140) permet ledit ajustement grossier, et ledit élément de manchon tournant permet l'ajustement fin de ladite fente.

18. Appareil selon les revendications 15, 16 ou 17 incluant un élément de bouton (144) à son extrémité proximale, ledit élément de bouton incluant un moyen (146) pour venir en prise avec ledit élément de loquet de telle sorte que ledit moyen d'engagement empêche la rotation dudit élément de manchon et dudit élément de bouton à moins que ledit élément de loquet soit en prise avec ledit élément de bouton.

19. Appareil selon l'une des revendications précédentes, où ledit moyen d'avance permet ledit ajustement fin avec une avance par incréments dudit élément d'enclume.

20. Appareil selon l'une des revendications 1 à 5, où ledit moyen d'avance (16) comporte un élément de saisie positionné à une extrémité proximale dudit appareil, ledit élément de saisie incluant un mécanisme à levier (200) apte à pivoter sur ledit élément de saisie le long d'un axe longitudinal dudit appareil pour faire avancer et pour rétracter ledit élément d'enclume.

21. Appareil selon la revendication 20, où ledit mécanisme à levier (200) est accouplé à un élément de tige intérieur (36) qui s'étend depuis un élément flexible (34) dans et qui est coaxial avec la portion de corps tubulaire (20) de telle sorte que le pivotement dudit mécanisme à levier fait avancer et rétracte ledit élément d'enclume.

22. Appareil selon la revendication 20 ou 21, où ledit moyen d'avance comporte en outre un ensemble à rochet (218, 220) pour faire avancer par incréments ledit élément d'enclume à la suite de l'avance dudit élément d'enclume par ledit pivotement dudit mécanisme de levier sur ledit élément de saisie le long d'un axe longitudinal dudit appareil.

23. Appareil selon la revendication 22, où ledit ensemble à rochet comporte une pluralité d'encoches (218) formant un élément de crémaillère sur une portion d'extrémité dudit élément de saisie et un positionneur formant un cliquet (220) sur une portion dudit mécanisme à levier, ledit positionneur et lesdites encoches coopérant pour réaliser une avance par incréments dudit élément d'enclume.
